## Europäisches Patentamt
## European Patent Office
## Office européen des brevets

(19)

(11) Veröffentlichungsnummer: **0 168 830**
**B1**

(12) **EUROPÄISCHE PATENTSCHRIFT**

(45) Veröffentlichungstag der Patentschrift: **11.04.90**

(51) Int. Cl.⁵: **A 61 K 39/395**, A 61 K 37/02, A 61 K 37/54, A 61 K 37/66

(21) Anmeldenummer: **85108962.3**

(22) Anmeldetag: **18.07.85**

(54) Eine Immunglobulinpräparation in Kombination mit einer anderen pharmakologisch wirksamen Präparation zur Verwendung bei der Behandlung von Krankheiten.

(30) Priorität: **20.07.84 DE 3426903**

(43) Veröffentlichungstag der Anmeldung:
**22.01.86 Patentblatt 86/04**

(45) Bekanntmachung des Hinweises auf die Patenterteilung:
**11.04.90 Patentblatt 90/15**

(84) Benannte Vertragsstaaten:
**AT BE CH DE FR GB IT LI LU NL SE**

(56) Entgegenhaltungen:
EP-A-0 035 204
DE-A-3 329 393
GB-A-1 564 666
US-A-4 412 990

(73) Patentinhaber: **Biotest Pharma GmbH**
**Flughafenstrasse 4**
**D-6000 Frankfurt 71 (DE)**

(72) Erfinder: **Stephan, Wolfgang, Dr.**
**P. Holzmannstrasse 84**
**D-6072 Dreieich (DE)**
Erfinder: **Dichtelmüller, Herbert, Dr.**
**Rossertstrasse 14**
**D-6231 Sulzbach/Ts. (DE)**
Erfinder: **Thrun, Alexander, Dr.**
**Konrad-Duden-Weg 39**
**D-6000 Frankfurt/Main (DE)**
Erfinder: **Schleussner, Hans, Dr.**
**Nasenring 26**
**D-6000 Frankfurt/Main (DE)**

(74) Vertreter: **Görtz, Dr. Fuchs, Dr. Luderschmidt**
**Patentanwälte**
**Abraham-Lincoln-Strasse 7 Postfach 46 60**
**D-6200 Wiesbaden (DE)**

EP 0 168 830 B1

Courier Press, Leamington Spa, England.

**Beschreibung**

Die Erfindung betrifft die Verwendung von Immunglobulinpräpartionen, die neben IgG noch andere Immunglobuline der Klassen IgM und IgA aufweisen, in Kombination mit einer weiteren, pharmakologisch wirksamen Präparation als Mittel bei der Behandlung von Krankheiten.

Die Verwendung von Immunglobulinen bei der Bekämpfung von Infektionen ist weit verbreitet. Bei resistenten Keimen ist die Anwendung von Immunglobulinen häufig die einzige erfolgversprechende Maßnahme.

Die bei kombinierter Anwendung von bekannten Immunglobulin-Präparationen und Antibiotika edzielbare synergistische Wirkung ist in zahlreichen Veröffentlichungen beschrieben worden. Normale Immunglobulin-Präparationen enthalten im wesentlichen das Immunglobulin IgG.

Auch über die gemeinsame Verwendung solcher Immunglobulin-Präparationen mit anderen pharmakologisch wirksamen Stoffen, wie beispielsweise Fibronectin ist bereits berichtet worden (Europäische Patentanmeldung 83105957.1).

In jüngster Zeit ist eine humane Immunglobulin-Präparation zur intravenösen Anwendung entwickelt worden, die als IGAM bezeichnet wird, und die neben dem Hauptbestandteil IgG zusätzlich die Immunglobuline der Klassen IgM und IgA enthält. IGAM wird aus der Cohn-Fraktion III gewonnen und besteht typischerweise aus etwa 3700 mg Ig G/100 ml einer 5 Gew.-%igen Immunglobulinlösung, etwa 600 mg IgM/100 ml einer solchen Lösung und etwa 820 mg IgA/100 ml einer solchen Lösung. Im allgemeinen kann IGAM betrachtet werden als ein Gemisch aus IgG, IgM und IgA, das etwa 65 bis 75 Gew.-% IgG, etwa 8 bis 20 Gew.-% IgM und etwa 8 bis 20 Gew.-% IgA, bezogen auf die Gesamtmenge Immunglobulin enthält. Die Herstellung von IGAM ist beschrieben in der DE—OS—29 01 822.

IGAM weist dank seines IgM-Gehaltes im Test der passiven Hämagglutination wesentlich (z.T. mehr als 10-fach höhere) Antikörpertiter auf als herkömmliche Immunglobulinpräparate, die nür IgG enthalten. Die Agglutination der Antigene (Bakterien) führt zum Binden der Infektionskeime (Klumpung), damit zu einer Verhinderung der Ausbreitung und einer schnelleren Erkennung un Beseitigung durch Antigen-vernichtende Immunzellen.

Über die pharmakologische Wirksamkeit von IGAM in kombinierter Anwendung mit anderen Heilmitteln ist bis jetzt nichts bekannt geworden. Es wurde nun überraschend gefunden, daß bei gemeinsamer Verwendung von IGAM mit bestimmten anderen pharmakologisch wirksamen Präparationen ein synergistischer Effekt eintritt. Diese Präparationen, die zusammen mit IGAM eine synergistische Wirkung zeigen, sind bestimmte Antibiotika, Tuftsin und andere Oligopeptide aus Immungloblinketten, Fibronectin, Interferone, $\alpha_1$-Antitrypsin, Antithrombin un Lysozym.

Die Erfindung wird nachstehend anhand von Ausführungsbeispielen näher erläutert.

IGAM wurde in allen Fällen als wässrige 5%-ige Immunglobulinlösung angewandt.

Die Kombinationspäräte können direkt in IGAM gelöst werden, wobei die Vorschriften für eine intravenöse Anwendung zu beachten sind.

Andererseits ist die Anwendung einer Mischung von IGAM mit der jewiligen Kombinationssubstanz in Pulverform möglich, wobie die Mischung von der Applikation gelöst wird.

Ebenson ist die Einarbeitung beider Substanzen in einen Träger, wie Tabletten oder Salbe möglich.

Die Anwendung der Kombination in der Therapie kann gleichzeitig, getrennt oder zeitlich gestaffelt erfolgen.

Tuftsin

Tuftsin ist ein Tetrapeptid, welches durch enzymatische Spaltung in der Milz aus dem Immunglobulin G freigesetzt wird und somit im Körper natürlicherweise vorkommt.

Durch Tuftsin werden eine Reihe immungoloigscher Prozesse angeregt, darunter die Aktivierung immunkompetenter Zellen (Steigerung der Phagozytose) und dadurch auch die beschleunigte Vernichtung der in den Organismus eingedrungenen Mikroorganismen.

Durch Immunglobuline werden diese Mikroorganismen einerseits gebunden, agglutiniert und für die Phagozytose vorbereitet. Durch Tuftsin wird die Phagozytose (Aufnahme der Mikroorganismen in die immunkompetenten Zellen und die anschließende Vernichtung) stimuliert. Dadurch unterstützen sich die beiden wesentlichen Prozesse der Vernichtung von Infektionserregern.

Mause wurden mit Pseudomonas aerruginosa ($10^7$/Tier intraperitoneal) infiziert. Danach wurde eine wässrige Lösung, die 0,5 mg Tuftsin in 0,5 ml IGAM-Lösung gelöst enthielt, intravenös appliziert. 25 ml wässrige Lösung wurde pro Körpergewicht appliziert. Nach 24 Stunden waren alle Tiere einer unbehandelten Kontrollgruppe verendet, ebenso alle Tiere, die nur Tuftsin erhielten. Durch die Gabe des Immunglobulinpräparats konnten 44% der Tiere geschützt werden, jedoch 67% der Tiere, die die Kombination von Immunglobulin und Tuftsin erhielten.

Auch künstlich hergestellte Oligopeptide können erfindungsgemäß zusammen mit den genannten Immunglobulinpräparationen verwendet werden, insbesondere zur Therapie von Infektionen und Mykosen.

Cefamandol

Cefamandol wurde stellvertretend für die Gruppe der Cephalosporin-Antibiotika ausgewählt.

In einem Experiment ähnlich dem mit Tuftsin wurden Mäuse (21 Tiere pro Gruppe) mit Salmonella typhimurium infiziert (i.p.), und danach mit Cefamandol, IGAM oder einer Mischung von IGAM und Cefamandol (pro Tier 5 mg Immunglobulin und 0,80 mg Cefamandol) intravenös therapiert. Das Überleben der Tiere wurde während 17 Tagen kontrolliert.

Ergebnis

In der unbehandelten Kontrollgruppe überlebten 23% der Tiere; durch Cefamandol allein wurden 32% der Tiere geschützt, durch IGAM alleine 50%. Durch die Kombination IGAM/Cefamandol wurden 78% der Tiere geschützt.

Moxalactam

Ein anderes Antibiotikum, Moxalactam, wurde in Kombination mit IGAM getestet. Eine injizierbare Lösung wurde zubereitet, indem 500 µg Moxalactam in 1 ml IGAM-Lösung gelöst wurden. In einem Versuch, der mit dem Versuch mit Cefamandol identisch war, wurde die gleiche Anzahl Mäuse mit Escherichia coli (i.p.) infiziert und danach intravenös mit der injizierbaren Lösung aus IGAM plus Moxalactam (20 ml Lösung pro kg Körpergewicht als Einzeldosis) behandelt. Die Ergebnisse waren identisch mit den für Cefamandol beschriebenen.

Fibronectin

Fibronectin ist eine Substanz, die natürlicherweise im Organismus vorkommt. Durch Fibronectin werden Bakterien zusammengeklebt, wodurch einerseits die Immunglobulin-bedingte Agglutination erleichtert wird, zum anderen einem frühzeitigen Verorauch der Immunglobuline vorgebeugt wird.

In einem entsprechend dem Versuch mit Tuftsin angelegten Experiment wurden Pseudomonas aeruginosa-infizierte Tierre mit einer Mischung von Fibronectin (250 units/Tier) und 25 mg IGAM therapiert. Die Schutzwirkung der Kombination war signifikant besser als die des Immunglobulins allein. Fibronectin allein zeigte keine Schutzwirkung.

In einem weiteren Versuch wurde die gleiche Anzahl Mäuse mit Staphylococus aureus infiziert und zunächst mit einer wässrigen Lösung behandelt, die 500 Einheiten Fibronectin/ml Lösung enthielt. Die applizierte Dosis betrug 10 000 Einheiten Fibronectin/kg Körpergewicht.

Danach wurde eine 5 Gew.-%ige Lösung von IGAM injiziert; die applizierte Dosis betrug 10 ml/kg Körpergewicht. Die beobachtete Schutzwirkung war noch besser als im Falle von Pseudomonas aeruginosa.

$\alpha_1$-Antitrypsin ($\alpha_1$ AT)

$\alpha_1$-AT ist ein Inhibitor de proteolytischen Aktivität. Diese proteolytische Aktivität wird durch bakterielle Infektion bedingt und verursacht u.a. die Entzündung am Ort bakterieller Infektion.

$\alpha_1$-Antitrypsin wirkt somit entzündungshemmend. Auch hier wird durch die Bindung (Agglutination) der Bakterien durch Immunglobuline und durch die entzündungshemmende Wirkung von $\alpha_1$-AT ein synergistischer Effekt hinsichtlich der therapeutischen Wirkung erzielt.

Anti-Thrombin III (AT III)

AT III hemmt die Bildung von Blutgerinseln (Thrombosen), welche einerseits durch Versperrung von Blutgefäßen die Verteilung der antiinfektösen Schutzproteine (Immunglobuline) hemmen, andererseits aber auch das Festsetzen von eingedrungenen Mikroorganismen begünstigen.

Bei versuchen ähnlich denen mit Tuftsin und cefamandol wurde eine synergistische Wirkung bei der gemeinsamen Anwendung von IGAM und Anti-Thrombin III beaobachtet.

Interferone

Interferone sind Botenstoffe, die eine benachbarte Zelle informieren, daß Viren in eine Zelle eingedrungen sind. Auf diese Botenstoffe reagiert die bedrohte Zelle dadurch, daß durch Umstellung bestimmter Stoffwechselwege die Vermehrung eines etwa eindringenden Virus gehemmt wird.

Interferone schützen somit ein Gewebe vor der Ausbreitung und dem Befall durch Viren.

Einerseits können Viren durch Immunglobuline gebunden und mit Hilfe von Komplement inaktiviert werden, andererseits durch Interferon die Schutzeinrichtungen benachbarter Zellen aktiviert werden; d.h., Viren auf der Suche nach geeigneten, zu befallenden Zellen können durch Immunglobuline neutralisiert, werden und zusätzlich werden die Zellen durch Interferon zur Virusabwehr angeregt Die synergistische Wirkung von Interferon und Immunglobulinen konnte festgestellt werden.

Lysozym

Lysozym kommt u.a. im Speichel oder der Eiflüssigkeit (Hühnerei) vor. Das Enzym spaltet in der bakteriellen Zellwand zwischen N-Acetylmuraminsäure und N-Acetylglucosamin. Auf diese Weise kann die Zellwandstruktur aufgelöst (Lyse) werden. Die Bruchstücke der auf diese Weise lysierten Baketrien können durch Immunglobuline abgefangen und der Elimination durch Immunzellen zugeführt werden.

Bei Experimenten ähnlich denen mit Tuftsin und Cefamandol wurde beoachtet, daß bei gemeinsamer Verwendung von IGAM und Lysozym eine synergistische Wirkung auftritt.

**Patentansprüche**

1. Immunglobulinpräparationen, die neben IgG noch andere Immunglobuline der Klassen IgM und IgA aufweisen, in Kombination mit einer weiteren pharmakologisch wirksamen Präparation zur Verwendung als Mittel bei der Behandlung von Krankheiten.

2. Kombination nach Anspruch 1 zur Behandlung von bakteriellen oder viralen Infektionen oder Mykosen.

3. Kombination nach Anspruch 1 oder 2, dadurch gekennzeichnet, daß die andere pharmakologisch wirksame Präparation ein Antibiotikum oder eine antiviral wirksame Substanz ist.

4. Kombination nach Anspruch 1 oder 2, dadurch gekennzeichnet, daß die andere pharmakologisch wirksame Präparation Tuftsin oder ein anderes Oligopeptid aus Immunglobulinketten ist.

5. Kombination nach Anspruch 1 oder 2, dadurch gekennzeichnet, daß die andere pharmakologisch wirksame Präparation Fibronectin ist.

6. Kombination nach Anspruch 1 oder 2, dadurch gekennzeichnet, daß die andere pharmakologisch wirksame Präparation ein Interferon, wie α, β- und insbesondere γ-Interferon ist.

7. Kombination nach Anspruch 1 oder 2, dadurch gekennzeichnet, daß die andere wirksame Präparation $\alpha_1$-Antitrypsin ist.

8. Kombination nach Anspruch 1 oder 2, dadurch gekennzeichnet, daß die andere pharmakologisch wirksame Präparation Antithrombin III ist.

9. Kombination nach Anspruch 1 oder 2, dadurch gekennzeichnet, daß die andere pharmakologisch wirksame Präparation Lysozym ist.

10. Erzeugnisse, welche Immunpräparationen enthalten, die neben IgG noch andere Immunglobuline der Klassen IgM und IgA aufweisen, und eine eine andere pharmakologisch wirksame Präparation als Kombinationspräparat zur gleichzeitigen, getrennten oder zeitlich abgestuften Anwendung in der Therapie.

11. Erzeugnisse nach Anspruch 10 zur Anwendung in der Therapie von bakteriellen oder viralen Infektionen oder von Mykosen.

12. Erzeugnisse nach Anspruch 10 oder 11, dadurch gekennzeichnet, daß diese andere pharmakologisch wirksame Präparation ein Antibiotikum oder eine antiviral wirksame Substanz ist.

13. Erzeugnisse nach Anspruch 10 oder 11, dadurch gekennzeichnet, daß die andere pharmakologisch wirksame Präparation Tuftsin oder ein anderes Oligopeptid aus Immunglobulin-ketten ist.

14. Erzeugnisse nach Anspruch 10 oder 11, dadurch gekennzeichnet, daß die andere pharmakologisch wirksame Präparation Fibronectin ist.

15. Erzeugnisse nach Anspruch 10 oder 11, dadurch gekennzeichnet, daß die andere pharmakologisch wirksame Präparation Interferon, wie α,β- und insbesondere γ-Interferon ist.

16. Erzeugnisse nach Anspruch 10 oder 11, dadurch gekennzeichnet, daß die andere pharmakologisch wirksame Präparation $\alpha_1$-Antitrypsin ist.

17. Erzeugnisse nach Anspruch 10 oder 11, dadurch gekennzeichnet, daß die andere pharmakologisch wirksame Präparation Antithrombin III ist.

18. Erzeugnisse nach Anspruch 10 oder 11, dadurch gekennzeichnet, daß die andere pharmakologisch wirksame Präparation Lysozym ist.

**Revendications**

1. Préparations d'immunoglobulines, qui contiennent en plus de l'IgG encore d'autres immunoglobulines des classes IgM et IgA, en combinaison avec une autre préparation pharmacologiquement active pour l'application dans le traitement de maladies.

2. Combinaison suivant la revendication 1, pour le traitement d'infections bactériennes ou virales ou de mycoses.

3. Combinaison suivant la revendication 1 ou 2, caractérisée en ce que l'autre préparation pharmacologiquement active est un antibiotique ou une substance à activité antivirale.

4. Combinaison suivant la revendication 1 ou 2, caractérisée en ce que l'autre préparation pharmacologiquement active est la tuftsine ou un autre oligopeptide issu de chaînes d'immunoglobulines.

5. Combinaison suivant la revendication 1 ou 2, caractérisée en ce que l'autre préparation pharmacologiquement active est la fibronectine.

6. Combinaison suivant la revendication 1 ou 2, caractérisée en ce que l'autre préparation pharmacologiquement active est un interféron, comme l'α-, le β- et, en particulier, le γ-interféron.

7. Combinaison suivant la revendication 1 ou 2, caractérisée en ce que l'autre préparation pharmacologiquement active est l'$\alpha_1$-antitrypsine.

8. Combinaison suivant la revendication 1 ou 2, caractérisée en ce que l'autre préparation pharmacologiquement active est l'antithrombine III.

9. Combinaison suivant la revendication 1 ou 2, caractérisée en ce que l'autre préparation pharmacologiquement active est le lysozyme.

10. Produits qui contiennent des immuno-préparations, qui comprennent en plus de l'IgG encore d'autres immunoglobulines des classes IgM et IgA, et une autre préparation pharmacologiquement active à l'état de préparation combinée pour l'administration simultanée, séparée ou échelonnée dans le temps en thérapeutique.

11. Produits suivant la revendication 10 pour l'administration dans la thérapeutique d'infections bactériennes ou virales ou de mycoses.

12. Produits suivant la revendication 10 ou 11, caractérisés en ce que l'autre préparation pharmacologiquement active est un antibiotique ou une substance à activité antivirale.

13. Produits suivant la revendication 10 ou 11, caractérisés ce ce que l'autre préparation pharmacologiquement active est la tuftsine ou un autre oligopeptide issu de chaînes d'immunoglobulines.

14. Produits suivant la revendication 10 ou 11, caractérisés en ce que l'autre préparation pharmacologiquement active est la fibronectine.

15. Produits suivant la revendication 10 ou 11, caractérisés en ce que l'autre préparation pharmacologiquement active est un interféron, comme l'α-, le β- et, en particulier, le γ-interféron.

16. Produits suivant la revendication 10 ou 11, caractérisés en ce que l'autre préparation pharmacologiquement active est l'$a_1$-antityrpsine.

17. Produits suivant la revendication 10 ou 11, caractérisés en ce que l'autre préparation pharmacologiquement active est l'antithrombine III.

18. Produits suivant la revendication 10 ou 11, caractérisés en ce que l'autre préparation pharmacologiquement active est le lysozyme.

**Claims**

1. Immunoglobulin preparations containing in addition to IgG, other immunoglobulins of classes IgM and IgA in combination with another pharmacologically effective preparation for use as a drug in the treatment of diseases.

2. A combination according to claim 1 for the treatment of bacterial or viral infections or mycoses.

3. A combination according to claims 1 or 2, characterized in that the other pharmacologically effective preparation is an antibiotic or a substance of antiviral effect.

4. A combination according to claims 1 or 2, characterized in that the other pharmacologically effective preparation is tuftsin or another oligopeptide of immunoglobulin chains.

5. A combination according to claims 1 or 2, characterized in that the other pharmacologically effective preparation is fibronectin.

6. A combination according to claims 1 or 2, characterized in that the other pharmacologically effective preparation is an interferon, such as an $a$-, $\beta$- and especially a $\gamma$-interferon.

7. A combination according to claims 1 or 2, characterized in that the other effective preparation is $a_1$-antitrypsin.

8. A combination according to claims 1 or 2, characterized in that the other pharmacologically effective preparation is antithrombin III.

9. A combination according to claims 1 or 2, characterized in that the other pharmacologically effective preparation is lysozyme.

10. Products containing immunopreparations comprising, in addition to IgG, other immunoglobulines of the IgM and IgA classes, and another pharmacologically effective preparation as a combination preparation for simultaneous, separate or timely staggered administration during therapy.

11. Products according to claim 10 for administration during the therapy of bacterial or viral infections or of mycoses.

12. Products according to claims 10 or 11, characterized in that the said other pharmacologically effective preparation is an antibiotic or a substance of antiviral effect.

13. Products according to claims 10 or 11, characterized in that the other pharmacologically effective substance is tuftin or another oligopeptide of immunoglobulin chains.

14. Products according to claims 10 or 11, characterized in that the other pharmacologically effective preparation is fibronectin.

15. Products according to claims 10 or 11, characterized in that the other pharmacologically effective preparation is interferon, such as $a$, $\beta$- and especially $\gamma$-interferon.

16. Products according to claims 10 or 11, characterized in that the other pharmacologically effective preparation is $a_1$-antitrypsin.

17. Products according to claims 10 or 11, characterized in that the other pharmacologically effective preparation is antithrombin III.

18. Products according to claims 10 or 11, characterized in that the other pharmacologically effective preparation is lysozyme.